(19)

**Europäisches Patentamt**

**European Patent Office**

**Office européen des brevets**

(11) **EP 4 582 534 A1**

(12) **EUROPEAN PATENT APPLICATION**
published in accordance with Art. 153(4) EPC

(43) Date of publication:
**09.07.2025 Bulletin 2025/28**

(21) Application number: **23860493.8**

(22) Date of filing: **31.08.2023**

(51) International Patent Classification (IPC):
*C12N 5/0735* $^{(2010.01)}$   *C12N 5/10* $^{(2006.01)}$

(52) Cooperative Patent Classification (CPC):
**C12N 5/06; C12N 5/10**

(86) International application number:
**PCT/JP2023/031853**

(87) International publication number:
**WO 2024/048731 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **31.08.2022   JP 2022138649**

(71) Applicant: **Kyoto University**
**Kyoto-shi, Kyoto 606-8501 (JP)**

(72) Inventors:
 • **SAITO, Hirohide**
  **Kyoto-shi, Kyoto 606-8501 (JP)**

 • **FUJITA, Yoshihiko**
  **Kyoto-shi, Kyoto 606-8501 (JP)**
 • **HIROSAWA, Moe**
  **Ube-shi, Yamaguchi 755-8505 (JP)**
 • **HAYASHI, Karin**
  **Kyoto-shi, Kyoto 606-8501 (JP)**
 • **TAKASHIMA, Yasuhiro**
  **Kyoto-shi, Kyoto 606-8501 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **METHOD FOR PRODUCING NAÏVE PLURIPOTENT STEM CELLS**

(57)     Naive pluripotent stem cells are produced at high efficiency. A method for producing naive pluripotent stem cells, wherein the method includes the following steps: (1) a step that cultures primed pluripotent stem cells in primed pluripotent stem cell culture medium; and (2) a step that cultures the cells obtained in step (1) in naïve pluripotent stem cell culture medium, including at least one of the following steps: (i) a step that introduces miRNA selected from miRNA belonging to the let-7 miRNA family and similar sequences thereto into the cells cultured in steps (1) or (2) or causes said miRNA to be expressed in said cells; or, a step that introduces an activity inhibitor of a let-7 miRNA family target protein into the cells cultured in steps (1) or (2) or causes said activity inhibitor to be expressed in said cells; and (ii) a step that introduces a bone morphogenetic protein into the cells cultured in steps (1) or (2), causes said bone morphogenetic protein to be expressed in said cells, or adds said bone morphogenetic protein to the medium of (1) or (2); or a step that adds a bone morphogenetic protein activator to the medium of steps (1) or (2).

**EP 4 582 534 A1**

**Description**

FIELD

The present invention relates to a method for producing naive pluripotent stem cells. More particularly, the invention relates to a method for producing naive pluripotent stem cells that are safe to transplant in a highly efficient manner from primed pluripotent stem cells.

BACKGROUND

Human iPS/ES cells are normally in a "primed" state resembling a post-implantation epiblast. However, it is thought that naive iPS/ES cells are in a more undifferentiated state, allowing them to differentiate into extraembryonic tissue such as placenta, for example. Naive iPS/ES cells are therefore expected to be useful for human early embryo research and transplant medicine. It is also thought that naive iPS/ES cells should be able to overcome the problem of differentiation resistance currently encountered with primed iPS cells.

It is known in the prior art that causing expression of the genes KLF2 and NANOG in primed pluripotent stem cells allows conversion from the primed state to the naive state (see NPL 1, for example). Methods have also been developed recently for converting primed pluripotent stem cells from the primed state to the naive state by addition of specified low molecular compounds (see NPLs 2 and 3, for example). Methods involving addition of low molecular compounds are known as "chemical resetting methods", and they offer advantages including lack of gene introduction, thus reducing the risk of genomic mutation and being more suitable for humans, as well as the convenience of requiring only addition to medium and of using low molecular compounds suitable for large-scale production, making such methods superior in terms of cost and availability. However, the methods disclosed in NPLs 2 and 3 necessitate long periods for a number of subculturing procedures to obtain naive cells, while their efficiency for producing naive cells has also been poor.

[CITATION LIST]

[NON PATENT LITERATURE]

[NPL 1] Takashima et al., Cell. 158,1254-1269 (2014).
[NPL 2] Guo et al., Development. 144, 2748-2763 (2017).
[NPL 3] Szczerbinska et al., Stem Cell Rep. 13, 612-626 (2019).

SUMMARY

[TECHNICAL PROBLEM]

Safer and more efficient methods for obtaining naive pluripotent stem cells will be required in the future, not only for the purpose of research but also for transplant medicine.

As a result of much diligent research, the present inventors have found a novel method that allows production of naive pluripotent stem cells without gene introduction and without using low molecular compounds such as valproic acid which have been used in chemical resetting methods of the prior art. It was further found that the substances used in the novel method also promote production of naive pluripotent stem cells in conventional chemical resetting methods. In addition, the inventors conceived of producing an increased number of naive pluripotent stem cell lines from primed pluripotent stem cells in a relatively short time period without the use of feeder cells, and the invention was thereupon completed.

Specifically, the invention encompasses the following.

[1] A method for producing a naive pluripotent stem cell, comprising the following steps:

(1) a step of culturing a primed pluripotent stem cell in culture medium for primed pluripotent stem cells; and
(2) a step of culturing the cell obtained in step (1) in culture medium for naive pluripotent stem cells,

and comprising either or both of the following steps:

(i) a step of introducing miRNA selected from among miRNA belonging to the let-7 miRNA family and their similar

2

sequences into the cell to be cultured in step (1) or (2), or expressing the miRNA in the cell; or

a step of introducing an activity inhibitor for a target protein of the let-7 miRNA family into the cell to be cultured in step (1) or (2), or expressing the activity inhibitor in the cell; and

(ii) a step of introducing bone morphogenetic protein into the cell to be cultured in step (1) or (2) and expressing the bone morphogenetic protein in the cell, or adding the bone morphogenetic protein to the medium of step (1) or (2); or

a step of adding a bone morphogenetic protein activator to the medium of step (1) or (2).

[2] The method according to [1] above, further comprising a step of adding Wnt inhibitor to the medium of step (1) or (2) either before or simultaneously with step (ii).

[3] The method according to [1] above, comprising (A) step (i), in which the culture medium for naive pluripotent stem cells of step (2) does not comprise a histone deacetylase inhibitor.

[4] The method according to [3] above, further comprising step (ii).

[5] The method according to [1] above, comprising (B) step (i) or step (ii), in which the culture medium for naive pluripotent stem cells of step (2) comprises a histone deacetylase inhibitor.

[6] The method according to [1] above, wherein the histone deacetylase inhibitor comprises valproic acid.

[7] The method according to [1] above, wherein in step (i), the miRNA belonging to the let-7 miRNA family or a similar sequence thereof is selected from among miR-98-5p, let-7a-5p, let-7b-5p, let-7c-5p, let-7e-5p, let-7f-5p, let-7g-5p, let-7i-5p, and miRNA having at least 80% sequence identity to the same.

[8] The method according to [1] above, wherein in step (ii), the bone morphogenetic protein is selected from among BMP2 and BMP4.

[9] The method according to [5] above, wherein in step (ii), the bone morphogenetic protein activator is selected from among SB-4 and isoliquiritigenin.

[10] The method according to [1] above, wherein in step (i), the target protein of miRNA belonging to the let-7 miRNA family is NR6A1 and/or TRIM71.

[11] The method according to [1] above, further comprising step (3) in which the cell obtained in step (2) is additionally cultured in culture medium for naive pluripotent stem cells containing Tankyrase/Wnt/β-catenin inhibitor and PKC inhibitor.

[12] The method according to [11] above, further comprising a step in which the cell obtained in step (3) is subcultured in culture medium for naive pluripotent stem cells according to [11].

[ADVANTAGEOUS EFFECTS OF INVENTION]

By using the method for producing naive pluripotent stem cells of the invention it is possible to produce naive pluripotent stem cells from primed pluripotent stem cells in a safe and rapid manner, without using feeder cells derived from mice. Moreover, naive conversion treatment for about 2 weeks is able to drastically increase the proportion of naive pluripotent stem cells to a maximum of 80% compared to the naive conversion efficiency of about 2% achieved by the chemical resetting methods known from publications such as NPLs 2 and 3. By the production method of the invention it is also possible to avoid steps that require gene introduction, allowing naive conversion to be promoted by using RNA molecules, proteins and low molecular compounds. This can contribute to furthering research on human early embryos. Moreover, since naive pluripotent stem cells are believed to be capable of differentiating to a wide range of cells such as placenta, it becomes possible to implement a wider range of cell therapies instead of the current cell therapies that use primed pluripotent stem cells.

BRIEF DESCRIPTION OF DRAWINGS

Fig. 1 is a graph showing the results of introducing an miRNA-responsive RNA OFF switch into primed and naive H9 EOS cells and analyzing the translation efficacy (TE) by flow cytometry. The dotted line indicated by the arrow is miR-98-5p, and the region enclosed by the dotted lines is the region with a difference in width decrease within $2^{(1.1)}$ for translation efficacy of the naive type as compared to the primed type.

Fig. 2 is a diagram illustrating a culture scheme for chemical resetting, in the method for producing naive cells of the Examples. Shown are a schematic diagram for H9EOS expressing naive cell-specific GFP, and the media used from Day 0 to Day 14.

Fig. 3 shows resetting efficiency with candidate miRNA mimics, for transfection of primed H9EOS with each candidate miRNA mimic on Day 1 of Fig. 2. The candidate miRNA mimics were miRNA mimics extracted as miRNA exhibiting efficacy difference of at least $2^{(1.1)}$ between the primed and naive states in Fig. 1, the "ctrl_mimic" was a non-response mimic, and "chemical" was a control without miRNA addition on Day 1 (chemical resetting alone).

Fig. 4 shows resetting efficiency for transfection of primed H9EOS with the similar sequence miR-98-5p.

Fig. 5 shows resetting efficiency for transfection of H9EOS with doxycycline inducing piggyBac vectors encoding 22 different genes at 13.6 ng/well for a total of 300 ng/well, and 200 ng/well of transposase (all mix), resetting efficiency for transfection of tagBFP (tagBFP) and resetting efficiency for no transfection (untransfected), on Day 1 of Fig. 2.

Fig. 6 shows values for resetting efficiency by transfection of H9EOS on Day 1 of Fig. 2 using 22 different vectors, with the exception of one each, standardized to resetting efficiency without transfection.

Fig. 7 shows resetting efficiency with different amounts of introduction of a plasmid vector encoding hBMP2 into H9EOS, on Day 1 of Fig. 2.

Fig. 8 is a set of phase contrast microscope images for introduction of an amount of 300 ng (top row) and 15 ng (bottom row) of plasmid vector encoding hBMP2 into H9EOS, on Day 2, Day 5, Day 7 and Day 14.

Fig. 9A is a set of phase contrast microscope images for introduction of an amount of 300 ng (top row) and 15 ng (bottom row) of plasmid vector encoding hBMP2 into H9EOS, on Day 1, Day 2, Day 3 and Day 5.

Fig. 9B is a set of phase contrast microscope images for introduction of an amount of 300 ng (top row) and 15 ng (bottom row) of plasmid vector encoding hBMP2 into H9EOS, on Day 7, Day 12 and Day 14.

Fig. 10 shows values for resetting efficiency of H9EOS with addition of SB-4, 4-hydroxychalcone (HyCh) and isoliquiritigenin (IsoLiq) at 1 $\mu$M, 5 $\mu$M or 10 $\mu$M each to the medium on Days 3-5 of Fig. 2, standardized to resetting efficiency of H9EOS without small molecule addition (Chemical only).

Fig. 11 shows a phase contrast microscope image (top left) and fluorescent image (top right) of H9EOS on Day 14, when SB-4 was added at 10 $\mu$M once per day to the medium on Days 3-5 of Fig. 2, and a phase contrast microscope image (bottom left) and fluorescent image (bottom right) of H9EOS on Day 14 without addition of SB-4 (Chemical only).

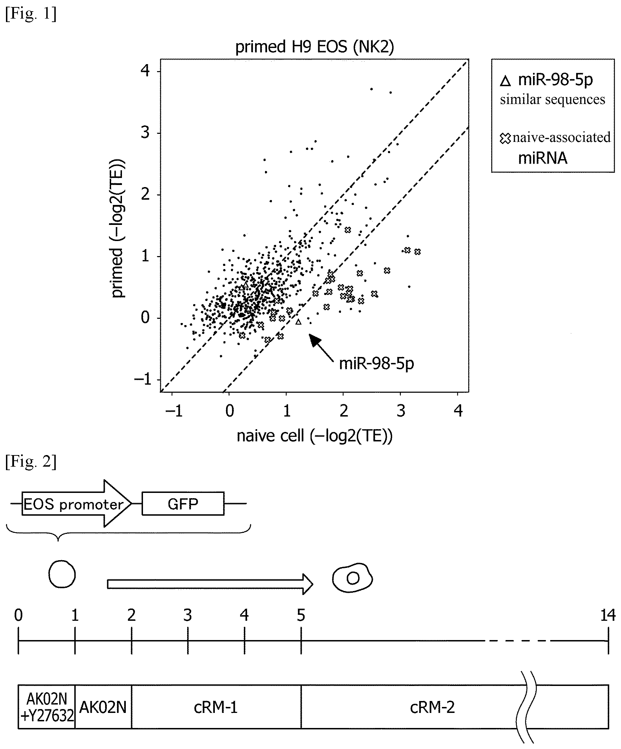

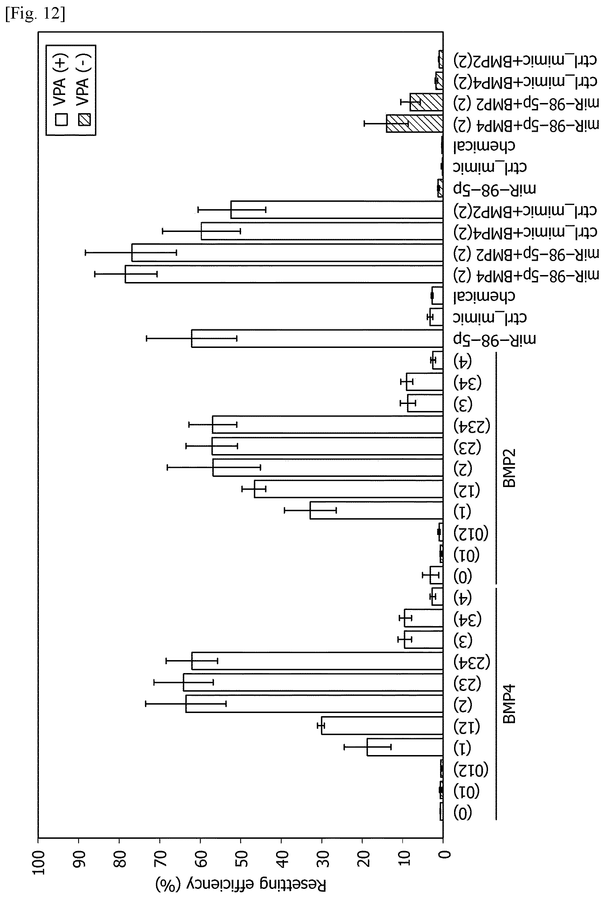

Fig. 12 is a graph showing differences in resetting efficiency with substances transfected into H9EOS, or substances added to medium and with different addition timings, shown for addition of the histone deacetylase (HDAC) inhibitor valproic acid (VPA) to cRM-1 (VPA+) and without addition (VPA-). The timing for addition of miR-98-5p or ctrl_mimic was on Day 1 of Fig. 2. The timing for addition of recombinant human bone morphogenetic protein (BMP2 (rhBMP2) or BMP4 (rhBMP4)) to medium was Day 0 (0), Day 1 (1), Day 2 (2), Day 3 (3) and/or Day 4 (4), where the numbers 0 to 4 in parentheses in the graph indicate addition of BMP over several times at the timing (Day) indicated by the numbers.

Fig. 13 shows phase contrast images (left panels) and fluorescent images (right panels) for H9EOS on Day 14, after transfection of miR-98-5p or ctrl_mimic into H9EOS on Day 1 of Fig. 2, and addition of rhBMP4 to the medium on Day 2.

Fig. 14 is a graph showing resetting efficiency as a percentage of EGFP positive cells, after transfection of H9EOS with siRNA for cell-intrinsic proteins targeted by the let-7 miRNA family, on Day 1 of Fig. 2. All siRNA represents transfection with all 20 types of siRNA, miR-98-5p and ctrl_mimic represent transfection of H9EOS with miR-98-5p or ctrl_mimic, respectively, on Day 1 of Fig. 2, and chemical represents no transfection with siRNA miR-98-5p, or ctrl_mimi. In all cases, the cells were cultured in the presence of VPA with exchange of VPA-containing medium on Day 2.

Fig. 15 shows results of detailed examination of NR6A1 siRNA, with resetting efficiency for transfection of H9EOS with miR-98-5p, ctrl_mimic and siRNA_NR6A1 on Day 1 of Fig. 2, and resetting efficiency with no addition (Chemical only). The experiment was conduct using a 24-well plate with n=3.

Fig. 16 is a graph showing resetting efficiency in culturing with VPA-containing cRM-1, using AdiPS iPS cells and H1, H9EOS and H9 ES cells.

Fig. 17 is a graph showing resetting efficiency with addition of BMP and/or Wnt inhibitor. The cells were cultured in the presence of VPA with exchange of VPA-containing cRM-1 on Day 2. The timing for addition of BMP and/or Wnt inhibitor to the medium was Day 0 (0), Day 1 (1) and/or Day 2 (2), where the numbers 0 to 2 in parentheses in the graph indicate addition of BMP and/or Wnt inhibitor to the medium over several times at the timing (Day) indicated by the numbers.

Fig. 18 is a graph showing resetting efficiency for addition of miRNA, siRNA or BMP on Day 1. The cells were cultured in the presence of VPA with exchange of VPA-containing cRM-1 on Day 2.

Fig. 19 is a graph showing resetting efficiency for addition of miRNA, siRNA and/or BMP on Day 1. The cells were cultured in the absence of VPA with exchange of VPA-free cRM-1 on Day 2.

DESCRIPTION OF EMBODIMENTS

Embodiments of the invention will now be explained. However, the present invention is not limited by the embodiments described below.

According to one embodiment, the invention relates to a method for producing naive pluripotent stem cells. The method for producing naive pluripotent stem cells comprises the following steps.

(1) A step of culturing primed pluripotent stem cells in culture medium for primed pluripotent stem cells; and
(2) a step of culturing the cells obtained in step (1) in culture medium for naive pluripotent stem cells not comprising histone deacetylase (HDAC) inhibitor or comprising HDAC inhibitor. Step (1) or (2) further comprises either or both of the following steps:

(i) a step of introducing miRNA selected from among miRNA belonging to the let-7 miRNA family and their similar sequences into the cells to be cultured in step (1) or (2), or expressing the miRNA in the cells; or
a step of introducing an activity inhibitor for a target protein of the let-7 miRNA family into the cells to be cultured in step (1) or (2), or expressing the activity inhibitor in the cells; and
(ii) a step of introducing bone morphogenetic protein into the cells to be cultured in step (1) or (2) and expressing the bone morphogenetic protein in the cells, or adding the bone morphogenetic protein to the medium of step (1) or (2); or

a step of adding a bone morphogenetic protein activator to the medium of step (1) or (2).

The naive pluripotent stem cells produced by the embodiment are pluripotent stem cells in a stable undifferentiated state known as the ground state, being also referred to as "reset cells". Naive pluripotent stem cells have the ability to differentiate into various types of cells including those of the placenta. Whether the produced cells are naive pluripotent stem cells, they can be confirmed by markers for naive pluripotent stem cells. Such markers include, but are not limited to SUSD2, Klf17, CD75 and Nanog. The cells can also be confirmed by qPCR and immunostaining. The resulting pluripotent stem cell cluster obtained by carrying out the steps of the invention also includes cells other than naive pluripotent stem cells.

Step (1)

The starting cells for the production method of the embodiment may be primed pluripotent stem cells. Especially, primed pluripotent stem cells may be primate pluripotent stem cells, which include but are not limited to, human, monkey, orangutan or chimpanzee pluripotent stem cells. Human pluripotent stem cells are preferred for purposes of producing grafting cells for regenerative medicine. For drug development or production of cells for experimentation, any desired primate pluripotent stem cells may be used. Primed pluripotent stem cells may be from somatic cells of humans or other primates, or cells from a biological secretion, where somatic cells include ordinary somatic cells, cells which may be in the blood, and cells which may be in the urine (renal tubular cells or urethra cells).
The pluripotent stem cells include embryonic stem (ES) cells, embryonic stem cells from a cloned embryo obtained by nuclear transfer (ntES), germline stem cells (GS cells), embryonic germ cells (EG cells) and induced pluripotent stem (iPS) cells and the like. They are preferably induced pluripotent stem (iPS) cells.
Primate pluripotent stem cells obtained by common methods of the prior art have been in the primed state. Whether the pluripotent stem cells used as starting cells are primed pluripotent stem cells, they can be confirmed by alkaline phosphatase staining or general markers for pluripotent stem cells. Examples of such markers include, but are not limited to, Nanog, Sox2, SSEA-1, SSEA-3, SSEA-4, TRA-1-60 and TRA-1-81 and the like. Population of primed pluripotent stem cells may be used for the invention even if they include naive pluripotent stem cells.
The culture medium for primed pluripotent stem cells used in step (1) may be any medium in which the primed pluripotent stem cells can proliferate while maintaining pluripotency. Such pluripotent stem cell culture medium may be medium containing bFGF (basic fibroblast growth factor) added to basal medium. Basal medium may be medium commonly used for culturing of animal cells, examples of which include BME medium, BGJb medium, CMRL 1066 medium, Glasgow's Minimal Essential Medium (GMEM), Improved MEM Zin Option medium IMDM medium, Medium 199 medium, Eagle MEM medium, αMEM medium, DMEM medium, F-12 medium, DMEM/F12 medium, IMDM/F12 medium, Ham's Medium, RPMI 1640 medium, Fischer's medium, Neurobasal medium, and mixed media of the same. For example, it is preferred to use basal medium further containing bFGF, the 20 essential amino acids, 10 vitamins (L-ascorbic acid, cobalamin, biotin, folic acid, i-inositol, niacinamide, D-calcium pantothenate, pyridoxine hydrochloride, riboflavin and thiamine hydrochloride) and 5 minerals (cupric sulfate, ferric sulfate, ferric nitrate, zinc sulfate and sodium selenite) (Scientific Reports, volume 4, Article number: 3594 (2014)). Especially, for production of naive pluripotent stem cells to be used for grafting into humans, the medium preferably containing no feeder cells. This is in order to avoid contamination by mouse-derived feeder cells. StemFit (registered trademark), AK02N and AK03N (Ajinomoto Co., Inc.) may be used as commercially available pluripotent stem cell culture medium suitable for feeder-free culturing, with no limitation to any specific medium.
A ROCK inhibitor may also be added to the culture medium for primed pluripotent stem cells to reduce cell death. ROCK inhibitors are not particularly restricted so long as they can inhibit the function of Rho-kinase (ROCK), and examples include Y-27632 (for example, see Ishizaki et al., Mol. Pharmacol. 57, 976-983(2000); Narumiya et al., Methods Enzymol.

325, 273-284(2000)), Fasudil/HA1077 (for example, see Uenata et al., Nature 389:990-994(1997)), H-1152 (for example, see Sasaki et al., Pharmacol. Ther. 93:225-232(2002)), Wf-536 (for example, see Nakajima et al., Cancer Chemother. Pharmacol. 52(4):319-324(2003)) and their derivatives, as well as antisense nucleic acid, RNA interference inducible nucleic acid (siRNA) and dominant negative mutants for ROCK, and their expression vectors. Other publicly known low molecular compounds may also be used as ROCK inhibitors (for example, see U.S. Patent Application Publication No. 2005/0209261, No. 2005/0192304, No. 2004/0014755, No. 2004/0002508, No. 2004/0002507, No. 2003/0125344, No. 2003/0087919, and International Patent Publication No. WO2003/062227, No. 2003/059913, No. 2003/062225, No. 2002/076976 and No. 2004/039796). One or two or more ROCK inhibitors may be used for the invention. One preferred ROCK inhibitor is Y-27632. The concentration of the ROCK inhibitor may be selected as appropriate by a person skilled in the art depending on the ROCK inhibitor used, and when Y-27632 is used as the ROCK inhibitor, for example, it may be 0.1 $\mu$M to 100 $\mu$M, preferably 1 $\mu$M to 50 $\mu$M and more preferably 5 $\mu$M to 20 $\mu$M. After culturing for a fixed period in ROCK inhibitor-containing medium, culturing is preferably continued for a fixed period in ROCK inhibitor-free medium.

Step (1) may be carried out over a period of about 1 to 4 days, for example, preferably about 1 to 3 days and more preferably about 2 days. According to the most preferred aspect, step (1) comprises, in order, a step of culturing for about 1 day in culture medium for primed pluripotent stem cells containing added ROCK inhibitor, and a step of culturing for about 1 day in culture medium for primed pluripotent stem cells without addition of ROCK inhibitor. However, the step of culturing for about 1 day in culture medium for primed pluripotent stem cells without addition of ROCK inhibitor may be omitted.

Step (2)

The cells obtained in step (1) are then cultured in culture medium for naive pluripotent stem cells either containing or not containing an HDAC inhibitor. A method of culturing in culture medium for naive pluripotent stem cells without an HDAC inhibitor in step (2) will be explained as "method (A)", and a method of culturing in culture medium for naive pluripotent stem cells containing an HDAC inhibitor in step (2) will be explained as "method (B)".

In methods (A) and (B), the culture medium for naive pluripotent stem cells used in step (2) is intended to include both induction medium used to induce naive pluripotent stem cells from the primed pluripotent stem cells, and maintenance medium for maintaining induced cells. Culture medium for naive pluripotent stem cells containing an HDAC inhibitor may be referred to herein as "naive pluripotent stem cell induction medium", and culture medium for naive pluripotent stem cells without an HDAC inhibitor may be referred to as "naive pluripotent stem cell maintenance medium".

The culture medium for naive pluripotent stem cells may be basal medium which comprises one or more compounds selected from among LIF (Leukemia inhibitory factor), MEK inhibitor, GSK3 inhibitor, cAMP production promoter, TGF-$\beta$ inhibitor, PKC inhibitor and Tankyrase/Wnt/$\beta$-catenin inhibitor. Naive medium preferably comprises LIF, in particular, and preferably comprises both LIF and GSK3 inhibitor. The LIF is preferably hLIF. The naive medium also preferably comprises essentially no bFGF. The LIF concentration in the medium is 1 ng/ml to 100 ng/ml or 5 ng/ml to 50 ng/ml, such as about 10 ng/ml. In step (2), preferably basal medium containing LIF and MEK inhibitor is used.

The basal medium for the culture medium for naive pluripotent stem cells is not particularly restricted, and examples include media prepared by addition of combinations of additives such as non-essential amino acids (NEAA), L-glutamine, 2-mercaptoethanol, antibiotics (such as streptomycin, penicillin, puromycin and mitomycin) and bovine serum albumin (BSA) and the like, to NDiff 227 medium (neural differentiation medium), N2B27 medium (1:1 mixed medium of N2 medium as N2 supplement added to DMEM/F12 medium and B27 medium as B27 supplement added to Neurobasal medium).

MEK inhibitors are not particularly restricted, and examples include PD0325901 (N-[(2R)-2,3-dihydroxypropoxy]-3,4-difluoro-2-[(2-fluoro-4-iodophenyl)amino]-benzamide; CAS Accession No.: 391210-10-9), U0126 (1,4-diamino-2,3-dicyano-1,4-bis[2-aminophenylthio]butadiene; CAS Accession No.: 109511-58-2), PD98059 (2-(2-amino-3-methoxyphenyl)-4H-1-benzopyran-4-one; CAS Accession No.: 167869-21-8), and PD184352 (2-(2-chloro-4-iodophenylamino)-N-cyclopropylmethoxy-3,4-difluorobenzamide; CAS Accession No.: 212631-79-3. PD0325901 is especially preferred among these. When PD0325901 is used as an MEK inhibitor, its concentration in the medium may be 50 nM to 100 $\mu$M or 100 nM to 10 $\mu$M, such as about 1 $\mu$M.

The GSK3 inhibitor is not particularly restricted, and examples include CHIR99021 (6-[[2-[[4-(2,4-dichlorophenyl)-5-(5-methyl-1H-imidazol-2-yl)-2-pyrimidinyl]amino]ethyl]amino]-3-pyridinecarbonitrile; CAS Accession No.: 252917-06-9), BIO (6-bromoindirubin-3'-oxime; CAS Accession No.: 667463-62-9), Kenpaullone (9-bromo-7,12-dihydroindolo[3,2-d][1]benzazepine-6(5H)-one; CAS Accession No.: 142273-20-9) and IM-16 (3-(4-fluorophenylethylamino)-1-methyl-4-(2-methyl-1H-indol-3-yl)-1H-pyrrole-2,5-dione; CAS Accession No.: 1129669-05-1). CHIR99021 is especially preferred among these. When CHIR99021 is used as a GSK3 inhibitor, its concentration in the medium may be 50 nM to 100 $\mu$M or 100 nM to 10 $\mu$M, such as about 1 $\mu$M.

The cAMP production promoter is not particularly restricted, and forskolin is one example. When forskolin is used as the cAMP production promoter, its concentration in the medium may be 50 nM to 100 $\mu$M or 100 nM to 10 $\mu$M, such as about 1 $\mu$M.

The TGF-β inhibitor is not particularly restricted, and examples include A83-01 (3-(6-methyl-2-pyridinyl)-N-phenyl-4-(4-quinolinyl)-1H-pyrazole-1-carbothioamide) and SB431542 (4-[4-(1,3-benzodioxol-5-yl)-5-(2-pyridinyl)-1H-imidazol-2-yl]-benzamide). A83-01 is especially preferred among these. When A83-01 is used as a TGF-β inhibitor, its concentration in the medium may be 10 nM to 100 μM or 100 nM to 10 μM, such as about 1 μM.

The PKC inhibitor is not particularly restricted, and examples include Go6983 (3-[1-[3-(dimethylamino)propyl]-5-methoxy-1H-indol-3-yl]-4-(1H-indol-3-yl)-1H-pyrrole-2,5-dione; CAS Accession No.: 133053-19-7) and GF109203X (3-(1-(3-dimethylamino)propyl)-1H-indol-3-yl)-4-(1H-indol-3-yl)-1H-pyrrole-2,5-dione; CAS Accession No.: 133052-90-1). Go6983 is especially preferred among these. When Go6983 is used as a PKC inhibitor, its concentration in the medium may be 50 nM to 100 μM or 100 nM to 10 μM, such as about 1 μM to 3 μM.

The Tankyrase/Wnt/β-catenin inhibitor is not particularly restricted, and examples include XAV939 (2-(4-trifluoromethylphenyl)-7,8-dihydro-5H-thiopyrano[4,3-d]pyrimidine-4(3H)-one; CAS Accession No.: 284028-89-3), IWT-2, IWP-3 and IWP-4. When XAV939 is used as a Tankyrase/Wnt/β-catenin inhibitor, its concentration in the medium may be 0.5 μM to 100 μM, such as about 2 μM.

The culture medium for naive pluripotent stem cells may be a commercially available medium, such as t2iLGo (N2B27 + PD0325901 (1 μM) + CHIR99021 (1 μM) + LIF + Go6983 (2-3 μM)), 5iLAF (N2B27 + PD0325901 (1 μM) + CHIR99021 (1 μM) + SB590885 (0.5 μM) + WH-4-023 (1 μM) + Y-27632 (10 μM) + LIF + Activin A), or tt2iLGo (N2B27 + PD0325901 (1 μM) + LIF + Go6983 (2 μM) + XAV939 (2 μM)).

The culture medium for naive pluripotent stem cells and the culture medium for primed pluripotent stem cells can be distinguished by the fact that the culture medium for primed pluripotent stem cells comprises essentially no LIF while the culture medium for naive pluripotent stem cells comprises essentially no bFGF.

In method (B), naive pluripotent stem cell induction medium comprising a histone deacetylase (HDAC) inhibitor is used. An "HDAC inhibitor" according to the invention is miRNA, siRNA, mRNA and/or a small molecule that inhibits histone deacetylase or suppresses its gene. Specific examples of HDAC inhibitors include, but are not limited to, valproic acid (VPA, alternate name: 2-Propylvaleric acid), sodium butyrate, suberoylanilide hydroxamic acid, BRD4884 (N-(4-amino-4'-fluoro[1,1'-biphenyl]-3-yl)tetrahydro-2H-pyran-4-carboxamide; CAS Accession No.: 1404559-91-6) and BRD6688 (N-[2-amino-5-(4-pyridinyl)phenyl]-1-pyrrolidinecarboxamide; CAS Accession No.: 1404562-17-9). When the HDAC inhibitor is a small molecule such as valproic acid, its concentration in the medium may be about 0.1 mM to 10 mM, and preferably about 0.5 mM to 5 mM.

The culturing using culture medium for naive pluripotent stem cells either comprising or not comprising an HDAC inhibitor in step (2) can be carried out for 1 to 5 days, for example, and preferably for 3 days. Method (A) for culturing in naive pluripotent stem cell maintenance culture medium without an HDAC inhibitor and method (B) for culturing in culture medium for naive pluripotent stem cells with an HDAC inhibitor will now be described in detail.

[(A) Production method without using an HDAC inhibitor]

Method (A) for production of naive pluripotent stem cells without adding an HDAC inhibitor to the medium of step (2) will now be described in detail. The method comprises the following steps.

(1) a step of culturing primed pluripotent stem cells in pluripotent stem cell culture medium; and
(2) a step of culturing the cells obtained in step (1) in culture medium for naive pluripotent stem cells comprising no HDAC inhibitor,

and additionally the following step (i):

(i) a step of introducing miRNA selected from among miRNA belonging to the let-7 miRNA family and their similar sequences into the cells to be cultured in step (1) or (2), or expressing the miRNA in the cells; or

a step of introducing an activity inhibitor for a target protein of the let-7 miRNA family into the cells to be cultured in step (1) or (2), or expressing the activity inhibitor in the cells.

The method may also optionally comprise the following step (ii) in addition to step (i).

(ii) A step of introducing bone morphogenetic protein into the cells to be cultured in step (1) or (2) and expressing the bone morphogenetic protein in the cells, or adding the bone morphogenetic protein to the medium of step (1) or (2); or a step of adding a bone morphogenetic protein activator to the medium of step (1) or (2).

For steps (1) and (2), as mentioned above, there is no need to add an HDAC inhibitor such as VPA to the culture medium for naive pluripotent stem cells used in (2).

In step (i), miRNA selected from among miRNA belonging to the let-7 miRNA family and their similar sequences is introduced into the cells to be cultured in step (1) or (2), or miRNA selected from among miRNA belonging to the let-7 miRNA family and their similar sequences is expressed in the cells to be cultured in step (1) or (2). There may also be included, in addition to or instead of this step, a step of introducing an activity inhibitor for a target protein of the let-7 miRNA family into the cells to be cultured in step (1) or (2), or expressing an activity inhibitor for a target protein of the let-7 miRNA family in the cells to be cultured in step (1) or (2).

Examples of miRNA belonging to the let-7 miRNA family include miR-98-5p, let-7a-5p, let-7b-5p, let-7c-5p, let-7e-5p, let-7f-5p, let-7g-5p and let-7i-5p. The sequences of these miRNA are shown in Table 1. Their similar sequences may also be used, and especially sequences with at least 80%, at least 85%, at least 90% or at least 95% sequence identity with their sequences, since they maintain identity of function with miRNA belonging to the let-7 miRNA family. One of these may be added alone, or two or more may be added in combination. The term "reset miRNA" may be used herein to refer to miRNA selected from among miRNA belonging to the let-7 miRNA family and their similar sequences. Table 1 shows specific examples of reset miRNA.

[Table 1]

| Sequence name | Sequence | SQID |
|---|---|---|
| miR-98-5p | UGAGGUAGUAAGUUGUAUUGUU | 1 |
| let-7a-5p | UGAGGUAGUAGGUUGUAUAGUU | 2 |
| let-7b-5p | UGAGGUAGUAGGUUGUGUGGUU | 3 |
| let-7c-5p | UGAGGUAGUAGGUUGUAUGGUU | 4 |
| let-7e-5p | UGAGGUAGGAGGUUGUAUAGUU | 5 |
| let-7f-5p | UGAGGUAOUAGAUUGUAUAGUU | 6 |
| let-7g-5p | UGAGGUAGUAGUUUGUACAGUU | 7 |
| let-7i-5p | UGAGGUAGUAGUUUGUGCUGUU | 8 |

The activity inhibitor for a target protein of the let-7 miRNA family may be a substance that suppresses expression of a target protein of the let-7 miRNA family, and/or that reduces activity of the target protein. The target protein of the let-7 miRNA family may be NR6A1 (Nuclear Receptor Subfamily 6 Group A Member 1), TRIM71 (Tripartite Motif Containing 71, alternate name: LIN41), IGF2BP1 (Insulin-like growth factor 2 mRNA-binding protein 1), VASH2 (Vasohibin-2) or DPH3 (Diphthamide Biosynthesis 3). Of these it is particularly preferred to suppress the activity of NR6A1 and/or TRIM71.

The activity inhibitor is not particularly restricted and may be a substance that can suppress expression of the protein by a knockdown method, or it may be a substance that can modify the gene encoding a protein by a knockout method. Substances used in knockdown methods in cells include siRNA for target proteins, and DNA constructs encoding shRNA. DNA constructs encoding shRNA are preferably plasmids encoding shRNA, and preferably plasmids encoding shRNA with drug-inducible expression. Substances used in knockout methods also include substances that knockout target proteins by CRISPR/Cas9-based genome editing technology. According to a preferred aspect, the activity inhibitor may be siRNA that is able to knockdown NR6A1, and/or siRNA that is able to knockdown TRIM71. For siRNA that is able to knockdown NR6A1, it may be a combination of several different siRNAs that can act on multiple sites of the NR6A1 gene, for example, such as a combination of 4 or more different siRNAs for the NR6A1 gene. Such siRNAs may be commercial products, or siRNAs may be appropriately designed and synthesized by a person skilled in the art based on the NR6A1 gene sequence. The same applies for TRIM71 as well.

The aforementioned reset miRNA, siRNA thereof capable of knockdown, and activity inhibitors for target proteins are sometimes collectively referred to as reset RNA.

Reset RNA can be artificially synthesized and introduced into cultured cells in the form of synthetic RNA molecules. RNA molecule introduction methods include, but are not limited to, lipofection methods, liposome methods, electroporation methods, calcium phosphate coprecipitation methods, DEAE-dextran methods, microinjection methods and gene gun methods and the like. The advantage of introducing synthetic RNA molecules is that they are not integrated into the genome, and the reset RNA-introduced cells can therefore easily be used for medical applications.

Reset RNA also can be added to cultured cells in the form of a DNA construct such as a vector, allowing the reset RNA to be biosynthesized in the cells. The expression vector encoding the sequence of the reset RNA may be a common one well-known in the field, such as a viral vector, artificial chromosome vector, plasmid vector or transposon expression system (also known as "transposon vector") and the like. Examples of viral vectors include retrovirus vectors, lentivirus vectors, adenovirus vectors, adeno-associated virus vectors and Sendai virus vectors. Examples of artificial chromosome vectors include human artificial chromosomes (HAC), yeast artificial chromosomes (YAC) and bacterial artificial chromosomes (BAC, PAC) and the like. A plasmid vector may be any common mammalian plasmid, such as an episomal vector.

Examples of transposon vectors include expression vectors using piggyBac transposon. The method of introducing the DNA construct may generally be the same as the method of introducing RNA molecules.

The period during which the reset RNA is introduced into the cultured cells or the reset RNA is expressed in cultured cells may be from 48 hours before carrying out the subsequent step (2) up to essentially simultaneously with exchange of the medium in step (2), and it is preferably from 48 hours up to 12 hours before step (2). When adding an RNA molecule composed of reset RNA, therefore, the RNA molecule may be introduced into the medium from 48 hours to 12 hours before carrying out the subsequent step (2). When a DNA construct encoding reset RNA is to be added, the DNA construct may be added to the medium between from 72 hours to 24 hours earlier. When the DNA construct is drug-induced shRNA, the DNA construct may be introduced into the cells beforehand, and the drug added at an arbitrary point to cause expression of the miRNA, siRNA or shRNA in the cultured cells. When step (1) does not comprise a step of culturing for about 1 day in culture medium for primed pluripotent stem cells without added ROCK inhibitor, preferably the cultured cells are transfected with the reset RNA at the stage of seeding the primed pluripotent stem cells into culture medium for primed pluripotent stem cells containing added ROCK inhibitor.

According to a preferred embodiment, when step (1) is to be carried out in order with a step of culturing for about 1 day in pluripotent stem cell culture medium containing added ROCK inhibitor (Day 0) and a step of culturing for about 1 day in pluripotent stem cell culture medium without added ROCK inhibitor (Day 1), the reset RNA is introduced into the cultured cells or the reset RNA is expressed in the cultured cells during the period from immediately after until about 5 hours after exchange with pluripotent stem cell culture medium without added ROCK inhibitor. The introduction in this case may be in an amount of 1 pmol to 10 pmol with respect to $8 \times 10^4$ cells, for example.

In method (A), at least step (i) may be carried out for introduction of at least the reset miRNA, siRNA or shRNA into the cells, to produce naive pluripotent stem cells from primed pluripotent stem cells without using an HDAC inhibitor.

The optional step (ii) comprises a step of introducing bone morphogenetic protein into the cells to be cultured in step (1) or (2) and expressing the bone morphogenetic protein in the cells, or adding the bone morphogenetic protein to the medium in step (1) or (2). In addition to or instead of these steps, it may also comprise a step of adding a bone morphogenetic protein activator to the medium of step (1) or (2).

Bone morphogenetic proteins (BMP) include, but are not limited to, BMP2, BMP4 and their analogs or derivatives. BMP may be preferably human BMP. The BMP to be added to the medium may also be a recombinant protein.

The method of introducing the BMP may be a method of directly transfecting the cells with a synthetic protein molecule, a method of introducing mRNA encoding BMP into the cells in the form of a synthetic RNA molecule, a method of introducing a DNA construct encoding BMP into the cells and expressing it in the cells, or a method of adding BMP to the medium. A method of directly transfecting cells with a synthetic protein molecule may employ any desired transfection reagent. Introduction of a synthetic RNA molecule or a DNA construct may be carried out in the same manner as the previous step (i).

The period for introducing, expressing or adding the BMP in the medium may be at any period during step (1) or (2). According to one embodiment, this may be within 1 to 3 days from the start of culturing, preferably within 24 hours from the start of culturing in step (2), and more preferably introduction, expression or addition of BMP in the medium is simultaneous with the start of step (2). Although the timing of introduction may differ depending on the form of the substance to be introduced, it is preferably within 24 hours from the start of culturing in step (2), with the BMP existing in an active state in the medium or in the cultured cells. The amount of introduction may be 5 ng/mL to 100 ng/mL, for example, and is preferably 5 ng/mL to 10 ng/mL.

According to another embodiment, introduction of BMP may be during any period in step (1), and within 24 hours from the start of culturing in step (2). When step (1) is carried out over a period of 2 days, in particular, it may be at any period up to 3 days from immediately after the start of culturing in step (1). The BMP may be introduced only once, or two or more times, during the period, and when introduced over several times, the BMP may be introduced at different stages in both step (1) and step (2).

When the BMP is introduced, expressed or added in step (1), preferably a Wnt inhibitor is also added to the medium either beforehand or simultaneously. The purpose of addition is to suppress induction of differentiation by BMP. When BMP is to be introduced at least once, and then introduced over several times, during the culturing of step (1), the Wnt inhibitor may be added before the first introduction, or simultaneously with the first introduction, or the Wnt inhibitor may be added during a later introduction, or not added at all. Specific examples of Wnt inhibitors may be the same options for Wnt inhibitors mentioned above as optional components for the culture medium for naive pluripotent stem cells. Particularly preferred are IWT-2, IWP-3 and IWP-4, and their arbitrary combinations, although there is no limitation to these. The amount of Wnt inhibitor added may be 0.1 $\mu$M to 100 $\mu$M, and preferably 0.25 $\mu$M to 5 $\mu$M. When the Wnt inhibitor is added over several times, the amount of addition still preferably satisfies the concentration specified by these ranges.

Even under conditions in which an HDAC inhibitor is not added to the medium, addition of BMP can improve the resetting efficiency approximately 7-fold compared to when BMP is not added.

In the step of adding a bone morphogenetic protein (BMP) activating substance to the medium of step (1) or (2), a substance that increases the activity of cell-intrinsic BMP or externally added BMP is added to the medium. The BMP activating substance may typically be a low molecular compound. Preferred BMP activating substances include, but are not limited to, SB-4 (CAS Accession No.: 100874-08-6) and isoliquiritigenin (CAS Accession No.: 961-29-5).

The BMP activating substance may be directly added to the medium of step (1) or (2). The timing of addition may be as desired, such as at the time of and in the same number of times of introduction or addition of BMP. According to one embodiment, the timing of addition is preferably within 1 to 3 days from the start of culturing in step (2), and may be more preferably 3 contiguous days after the start of step (2). When the BMP activating substance is added to the medium of step (1), preferably a Wnt inhibitor is also added to the medium either beforehand or simultaneously. The timing of and number of additions of Wnt inhibitor may be the same as for introduction and addition of BMP. The amount of BMP activating substance introduced in this case may be 7 $\mu$M to 50 $\mu$M, for example. By carrying out a step of adding a BMP activating substance, it is possible to significantly increase production efficiency for naive pluripotent stem cells, and to increase production volume of naive pluripotent stem cells compared to the prior art, during a shorter culturing period.

The BMP introduction step or the BMP activating substance addition step may each be carried out alone, or both may be carried out together. Step (ii) may be a step of activating the BMP signal transduction pathway in cells. Carrying out such these steps can activate the cellular BMP signal transduction pathway and improve the resetting efficiency.

In method (A), naive pluripotent stem cells can be produced by introducing reset RNA and optionally BMP or a BMP activating substance into cells at a predetermined timing during the culturing step, essentially without adding an HDAC inhibitor, and especially VPA, to the medium. Using an embodiment in which both reset RNA and BMP or a BMP activating substance are introduced can increase production volume of naive pluripotent stem cells by about 2-fold to about 20-fold in a shorter culturing period, compared to conventional culturing using an HDAC inhibitor such as VPA, without using reset RNA and without using BMP or a BMP activating substance. For conventional production of naive pluripotent stem cells it has been considered essential to introduction KLF2 and NANOG genes, or to carry out culturing in the presence of a low molecular compound HDAC inhibitor such as VPA. However, method (A) has the advantage of allow naive pluripotent stem cells to be produced inexpensively and efficiently without using an HDAC inhibitor.

[(B) Production method using HDAC inhibitor]

Method (B) for production of naive pluripotent stem cells without adding an HDAC inhibitor to the medium will now be described in detail. The method comprises the following steps.

(1) a step of culturing primed pluripotent stem cells in pluripotent stem cell culture medium; and
(2) a step of culturing the cells obtained in step (1) in culture medium for naive pluripotent stem cells containing an HDAC inhibitor,

and comprising either of the following steps (i) and (ii):

(i) a step of introducing miRNA selected from among miRNA belonging to the let-7 miRNA family and their similar sequences into the cells to be cultured in step (1) or (2), or expressing the miRNA in the cells; or
a step of introducing an activity inhibitor for a target protein of the let-7 miRNA family into the cells to be cultured in step (1) or (2), or expressing the activity inhibitor in the cells; and
(ii) a step of introducing bone morphogenetic protein into the cells to be cultured in step (1) or (2) and expressing the bone morphogenetic protein in the cells, or adding the bone morphogenetic protein to the medium of step (1) or (2); or

a step of adding a bone morphogenetic protein activator to the medium of step (1) or (2).

The method may also optionally comprise both steps (i) and (ii).

Step (i) of method (B) can be carried out in the same manner as step (i) of method (A), introducing either or both reset miRNA and/or a target protein activity inhibitor into the cells.

Even if the step of introducing reset miRNA in step (i) is carried out alone without carrying out step (ii) in method (B), it is still possible to significantly improve production efficiency for naive pluripotent stem cells, and to increase the production volume of naive pluripotent stem cells by at least 2-fold to nearly 20-fold over the prior art, with a shorter culturing period. Moreover, even if the step of introducing a target protein activity inhibitor such as siRNA in step (i) is carried out alone without carrying out step (ii), it is still possible to significantly improve production efficiency for naive pluripotent stem cells, and to increase the production volume of naive pluripotent stem cells compared to the prior art, with a shorter culturing period.

Step (ii) in method (B) may be carried out in the same manner as step (ii) of method (A), carrying out either or both the step of introducing BMP into the cells or adding it to the medium, and/or the step of adding a BMP activating substance to the medium. Even if the BMP-introduction step of step (ii) is carried out alone without carrying out step (i), it is still possible to significantly improve production efficiency for naive pluripotent stem cells, and to increase the production volume of naive pluripotent stem cells by about 24-fold, and with a shorter culturing period, compared to conventional chemical resetting methods carried out in medium containing VPA and without using bone morphogenetic protein. Moreover, even if the step of adding a BMP activating substance in step (ii) is carried out alone without carrying out step (i), it is still possible to significantly improve production efficiency for naive pluripotent stem cells, and to increase the production volume of naive pluripotent stem cells by about 3-fold compared to the prior art, with a shorter culturing period.

Steps (i) and (ii) of method (B) may also be carried out in combination. Combining steps (i) and (ii) can potentially increase production volume by about from 2-fold to 20-fold compared to when these steps are not carried out.

For both (A) the production method without using an HDAC inhibitor and (B) the production method using an HDAC inhibitor, step (1) or (2) may be further followed by an optional step (3) in which the cells obtained in step (2) are additionally cultured in culture medium for naive pluripotent stem cells without an HDAC inhibitor (naive pluripotent stem cell maintenance medium). The naive pluripotent stem cell maintenance medium used in step (3) preferably contains LIF, a MEK inhibitor, a Tankyrase/Wnt/$\beta$-catenin inhibitor and a PKC inhibitor in basal medium. More specifically, it preferably contains hLIF, PD0325901, XAV939 and Go6983. The naive pluripotent stem cell maintenance medium used in step (3) essentially contains no HDAC inhibitor such as VPA.

Step (3) may be carried out for 8 days to 130 days, for example, and particularly for 9 days. It was observed that carrying out step (3) promoted conversion to the naive state by Day 5 and formation of a naive-like cell mass by Day 14, suggesting that continued culturing would lead to further maturation of naive cells.

The total culturing period for steps (1) and (2) and the optional step (3) may be at least 14 days, and a maximum of about 135 days, such as about 25 to 30 days, and most preferably 28 days.

The aforementioned step may further comprise an additional step of subculturing the cells obtained in step (2) or step (3). Subculturing may consist of culturing for 1 up to about 10, 20 or more subcultures using culture medium for naive pluripotent stem cells containing no HDAC inhibitor (naive pluripotent stem cell maintenance medium). This can yield a cell population with the content ratio of naive pluripotent stem cells increased by about 90%.

EXAMPLES

The invention will now be explained in greater detail by Examples. However, the Examples are not intended to restrict the scope of the invention.

[Experiment method]

[Activity measurement using miRNA-responsive OFF switch library]

· Preparation of miRNA-responsive OFF switch library

Synthetic oligo DNA containing T7 promoter (KEC879), synthetic oligo DNA containing a sequence complementary to miRNA (723 types), TagBFP ORF and reverse primer including poly-T (KEC883) were amplified by PCR using TOYOBO KOD Plus Neo polymerase to prepare a library template. The template was purified using SpeedBeads magnetic carboxylate modified particles or Monarch DNA Cleanup Columns and a MinElute Reaction Cleanup Kit. The mRNA used as an internal standard (reference) was prepared by PCR amplification of synthetic oligo DNA containing the UTR sequence (KEC001, KEC062, KEC063, KEC065), hmAG1 ORF and reverse primer (KEC883) containing poly-T, using TOYOBO KOD Plus Neo polymerase. Preparation was also with synthetic oligo DNA (YF0771) containing T7 promoter and UTR and a reverse primer (KEC883) containing poly-T, on a plasmid obtained by cloning UTR and hmAG1 in pUC19 vector. The primers used for the experiment are shown in Table 2.

[Table 2]

| Sequence name | Sequence | SQID |
|---|---|---|
| KEC001_ Rev5UTR | CATGGTGGCGACCGGTGTCTTATATTTCTTCTTACTC | 9 |
| KEC062_1 VT_5prim c_UTR | CAGTGAATTGTAATACGACTCACTATAGGGCGAATTAAGAGAGAA AAGAAGAGTAAGAAGAAATATAAGACACCGGTCGCCACCATG | 10 |
| KEC876_ TAP_T7_1 G | CAGTGAATTGTAATACGACTCACTATAG | 11 |

(continued)

| Sequence name | Sequence | SQID |
|---|---|---|
| KEC063_1 VT_3prime .UTR | TCTAGACCTTCTGCGGGGCTTGCCTTCTGGCCATGCCCTTCTTCT CTCCCTTGCACCTGTACCTCTTGGTCTTTGAATAAAGCCTGAGTA GG | 12 |
| K EC065_ Re-v3UTR 2T20 | TTTTTTTTTTTTTTTTTTTTCCTACTCAGGCTTTATTCAAAGACCA AG | 13 |
| KEC883_ 3UTR120 A | TTTTTTTTTTTTTTTTTTTTTTTTTTTTTTTTTTTTTTTTTTTTTTTTT TTTTTTTTTTTTTTTTTTTTTTTTTTTTTTTTTTTTTTTTTTTTTTTTT TTTTTTTTTTTTTTTTTTTTTTTTTCCTACTCAGGCTTTATTCA | 14 |
| YF0771-T 7_5UTR_f wd | ATTGTAATACGACTCACTATAGGGCGAATTAAGAGAGAAAAGAAG AGTAAG | 15 |

The mRNA synthesis was carried out using a MEGAscript T7 Transcription Kit (Catalog No. AMB13345) by Thermo Fisher Scientific. For transcription, pseudouridine-5'-triphosphate (TriLink, N-1019-10, Ψ) and 5-methylcytidine-5'-triphosphate (TriLink, N-1014-10) were used instead of UTP and CTP, respectively. GTP and CAP analogs (TriLink, N-7003-10, ARCA) were also combined at 1:4 and added to the Cap structure. After transcription, the template was digested with TURBO DNase (Thermo Fisher Scientific, AM2238), and following dephosphorylation, SpeedBeads magnetic carboxylate modified particles, RNAClean XP (Beckman Coulter, A63987) or a FavorPrep total RNA extraction column (FAVORGEN, FARBC-C50) was used for mRNA purification.

Introduction and fluorescence measurement of miRNA-responsive OFF switch

For the H9 cells there were used a stable line (H9EOS (NK)) having GFP with EOS (Early Transposon promoter and Oct-4 (Pou5f1) and Sox2 enhancers) upstream, and doxycycline-inducible Klf2 and Nanog, inserted into the genome. Primed and naive state H9EOS (NK) cells were seeded in a 96 well plate, and on the following day an miRNA-responsive OFF switch and mRNA encoding hmAG1 fluorescent protein as an internal standard were cotransfected using Lipofectamine MessengerMAX Transfection Reagent (Thermo Fisher Scientific, LMRNA015). On the day following transfection, Accumax(Nacalai Tesque, 17087-54) was added prior to incubation at 37°C, and the cells were separated from the plate and analyzed by flow cytometry (LSRFortessa, BD). The value obtained by dividing the tagBFP fluorescence of the switch by the internal standard hmAG1 was standardized by the value of the tagBFP fluorescence of normal tagBFP mRNA divided by the fluorescence of the internal standard hmAG1, to quantify the miRNA activity in the cells. Candidate miRNA was selected as miRNA having activity of at least $2^{1.1}$ in naive state cells compared to primed cells.

[Cell culturing]

The H9EOS, H9EOS, H9 and H1 ES cells and the AdiPS iPS cells were cultured under Feeder-free conditions with AK02N (Ajinomoto, AK02N), and maintained with exchange of medium every few days. Subculturing was carried out by rinsing once with PBS, subsequently adding Accutase (Fujifilm, 12679-54) and incubating at 37°C for about 10 minutes, and then detaching the cells by pipetting. After centrifuging the cell suspension, the supernatant was removed and the cells were resuspended in AK02N, after which the cell count was measured using a Countess Automated Cell Counter and the cells were seeded at 16,000-30,000 cells/well in AK02N medium containing Y-27632 (Nacalai Tesque, 08945-84) and iMatrix-511 silk (Nippi, 387-10131), and subcultured.

[Chemical resetting]

After collecting H9EOS and H9EOS (NK) cells and measuring the cell count in the same manner as for subculturing, they were diluted with AK02N medium containing Y-27632 (Nacalai Tesque, 08945-84) and iMatrix-511 silk (Nippi, 387-10131) (culture medium for primed pluripotent stem cells) and seeded on a multiwell plate (modified method of G. Guo, et. al., 144, 2748-2763(2017)). The medium was exchanged with AK02N on Day 1, with culturing in cRM-1 (NDiff 227 (Takara, Y40002) containing 1 μM PD0325901, 10 ng/mL hLIF, 1 mM VPA) or VPA-free cRM-1 (culture medium for naive pluripotent stem cells) from Day 2 to Day 5, followed by culturing up to Day 14 in cRM-2 (NDiff 227

containing 1 μM PD0325901, 10 ng/mL hLIF, 2 μM Go6983, 2 μM XAV939). The resetting efficiency was quantified by counting the cell count using a flow cytometer, with the EGFP-expressing cells as naive cells. The results are summarized in Fig. 2.

[Improved resetting efficiency with miRNA]

The mimic used for each miRNA was mirVana miRNA Mimic (Thermo Fisher Scientific). A control, mirVana miRNA Mimic, Negative Control (Thermo Fisher Scientific, 4464058) was also used. Introduction was carried out on either Day 1, 2, 3 or 4, or a combination thereof, during the normal course of resetting, using Lipofectamine RNAiMAX Transfection Reagent (Thermo Fisher Scientific, 13778075) as the miRNA mimic. Culturing was then carried out according to common resetting protocol, and the EGFP-positive cells were quantified on Day 14 using a flow cytometer.

[scRNA-seq]

Single cell RNA-seq was conducted using Chromium Next GEM Single cell 3'-Library & Gel Beads Kit v3.1 for Single Index (10 x Genomics, 1000128), according to the manufacturer's protocol. The H9EOS cells transfected with the miRNA mimic (miR-98-5p or Negative control) on Day 1 were collected on Day 3, Day 8 and Day 14. The cells were rinsed twice with PBS, after which Accumax was added prior to incubation for 15 min, and the cells were detached by pipetting and suspended in PBS (0.04% BSA). After centrifuging at 300 x g, 5 min, the supernatant was removed and the cells were slowly suspended again in PBS (0.04% BSA) before repeating rinsing 2 to 3 times. The cell count in the cell suspension was measured with a Countess, and the cells were diluted to a target cell recovery of 5,000 cells and used in scRNA-seq. For the library, the average size was measured using Agilent High Sensitivity DNA kit (Agilent, 5067-4626) or QSep and the concentration was measured using a Qubit dsDNA HS Assay Kit (Thermo Fisher Scientific, Q32851). The library was sequenced using NextSeq 500 (Illumina) or NovaSeq (Illumina). Analysis was performed using Cell Ranger, and genes were selected that specifically increased or decreased expression levels in the miR-98-5p- introduced cells.

[Construction of plasmids]

On Day 3 after initial resetting, the total RNA of the cell was collected using a mirVana miRNA Isolation Kit (Thermo Fisher Scientific, AM1561). A ReverTraAce qPCR RT Master Mix (Toyobo, FSQ-201) was used to construct cDNA from the total RNA. Genes with increased expression level by miR-98-5p were amplified from cDNA by PCR using KOD Plus Net DNA polymerase, and then cloned in doxycycline-inducible piggyBac vector (pPB-TRE2-PshAI-HygR) using an InFusion cloning kit (Takara, Z9650N). The sequences were confirmed using a sequencer, and the plasmids were purified with a PureYield Plasmid Midiprep System (Promega, A2495). A total of 22 forced expression vectors were prepared: pPB-TRE2-ANGPT1-HygR, pPB-TRE2-ANXA2-HygR, pPB-TRE2-BAMBI-HygR, pPB-TRE2-BMP2-HygR, pPB-TRE2-FTL-HygR, pPB-TRE2-GATA2-HygR, pPB-TRE2-HPGD-HygR, pPB-TRE2-IDI-HygR, pPB-TRE2-ID3-HygR, pPB-TRE2-LAYN-variant1-HygR, pPB-TRE2-PCAT14-HygR, pPB-TRE2-PCDH17-HygR, pPB-TRE2-SHISA9-HygR, pPB-TRE2-SLC6A8-HygR, pPB-TRE2-SLC7A3-HygR, pPB-TRE2-SOX2-OT-HygR, pPB-TRE2-SOX4-HygR, pPB-TRE2-SPP1-HygR, pPB-TRE2-TAGLN-HygR, pPB-TRE2-TFAP2A-HygR, pPB-TRE2-TWIST1-HygR and pPB-TRE2-VIM-HygR.

[Improved resetting efficiency with plasmids]

On Day 1 of the resetting protocol shown in Fig. 2, the 22 plasmids were used, either alone or as a mixture of the 22 plasmids minus one each, for transfection together with the transposase-expressing plasmid (pCAG-PBase), using Lipofectamine Stem Transfection Reagent (Thermo Fisher Scientific, STEM00003). Doxycycline was added at a final concentration of 250 ng/mL to cRM-1 on Days 2 to 5 to induce gene expression. Culturing was then carried out according to common resetting protocol, and the EGFP-positive cells were quantified on Day 14 using a flow cytometer.

[Improved resetting efficiency by small molecule BMP activator]

SB-4 (Tocris, 6881/50), 4-Hydroxychalcone (FujiFilm-Wako Chemicals, 320-75521) or Isoliquirltigenin (Fluorochem, 462708) was added to cRM-1 containing VPA on Days 2 to 5 of the resetting protocol of Fig. 2. Culturing was then carried out according to common resetting protocol, and the EGFP-positive cells were quantified on Day 14 using a flow cytometer.

[Improved resetting efficiency with rhBMP4]

To cRM-1 containing or not containing VPA there was added 10-100 ng/mL of rhBMP4 (Reprocell, 314-BP-010), on one or more of Days 1 to 5 of the resetting protocol of Fig. 2. Culturing was then carried out according to common resetting protocol, and the EGFP-positive cells were quantified on Day 14 using a flow cytometer.

[Improved resetting efficiency with siRNA]

On Day 1 of the resetting protocol of Fig. 2, one siRNA from among NR6A1, TRIM71, LRIG1, DNMT3B, IGF2BP1, CACHD1, PCDH8, L1TD1, SFRP1, VASH2, LIN28B, USP44, MAPK10, DICER1, DPH3, PRTG, MAPK6, HMGA2, LINC01356 (n491112) and LINC01356 (n549768) (all purchased from Horizon Discovery/Dharmacon) was introduced using Lipofectamine RNAiMAX Transfection Reagent (Thermo Fisher Scientific, 13778075). Culturing was then carried out according to common resetting protocol, and the EGFP-positive cells were quantified on Day 14 using a flow cytometer.

[SUSD2 staining protocol]

AccuMax was added to the cells on Day 14 of the resetting protocol of Fig. 2, and the mixture was incubated at 37°C for about 10 to 15 minutes, after which the cells were detached. They were then suspended in 1% BSA-PBS to prevent nonspecific antibody binding, and after 30 minutes of blocking on ice, SUSD2 antibody was mixed with the cell suspension to a final concentration of 1:500, reaction was conducted for 30 minutes on ice and the mixture was rinsed with PBS, after which measurement was performed with a flow cytometer.

[Results]

Fig. 1 is a graph showing the results of introducing an miRNA-responsive RNA OFF switch into primed and naive H9 EOS (NK2) cells and analyzing the translation efficacy (TE) by flow cytometry. The translation efficacy TE was determined by the following formula.

$$TE = (Switch\ tagBFP/hmAG1)/(Ctrl\ tagBFP/hmAG1)$$

Switch tagBFP is the expression level of tagBFP with the miRNA-responsive RNA OFF switch encoding tagBFP, hmAG1 is the expression level of hmAG1 from mRNA encoding hmAG1 without the miRNA target site, and Ctrl tagBFP is the expression level of tagBFP from mRNA encoding tagBFP without the miRNA target site. The value of Switch tagBFP/hmAG1 and the value of Ctrl tagBFP/hmAG1 were measured using a flow cytometer. As shown in Fig. 1, several novel miRNAs with high activity in naive cells were also expressed in addition to miRNA (naive-associated miRNA) known by previous reports (mRNA in bottom right region of Fig. 1).

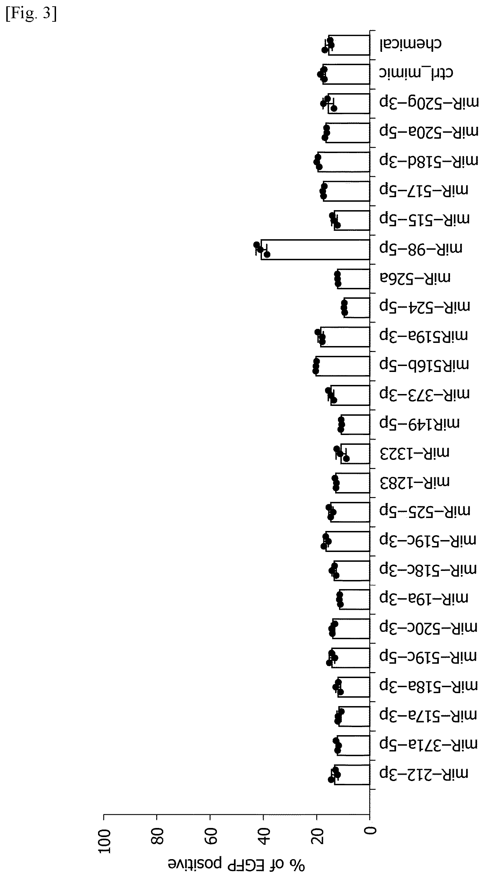

Fig. 3 shows resetting efficiency with candidate miRNA mimics, for transfection of primed H9EOS with each candidate miRNA mimic on Day 1 of Fig. 2, with cRM-1 containing VPA, in terms of EGFP-positive cells. Fig. 3 confirmed that the resetting efficiency of miR-98-5p is significantly high among the candidate miRNA mimics. The "chemical" in Fig. 3 represents resetting efficiency when culturing was carried out in VPA-containing cRM-1 without transfection with miRNA mimic or ctrl_mimic.

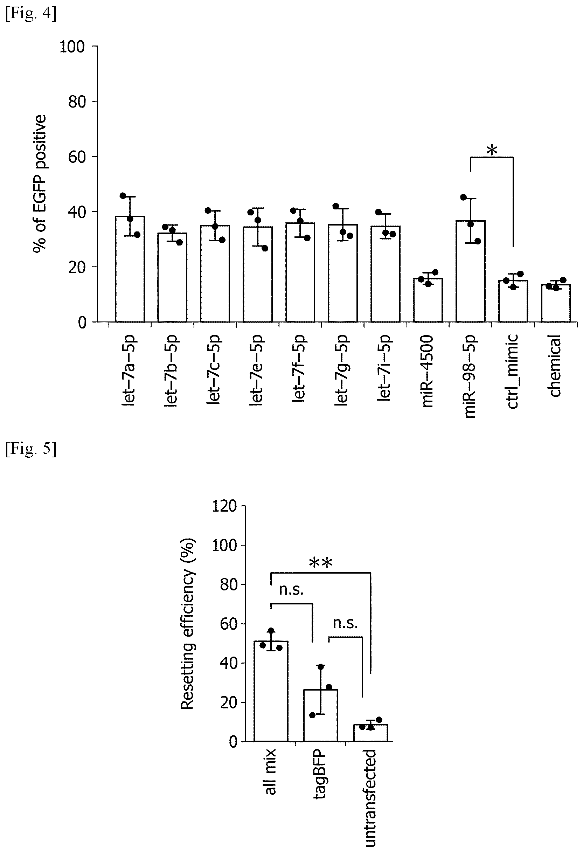

Fig. 4 shows resetting efficiency for transfection of primed H9EOS with similar sequences for miR-98-5p on Day 1 of Fig. 2, when VPA was added to cRM-1. The similar sequences used were let-7a-5p, let-7b-5p, let-7c-5p, let-7e-5p, let-7f-5p, let-7g-5p, let-7i-5p and miR-4500. Fig. 4 shows that sequences wherein the seed sequence had 100% matching with miR-98-5p exhibited high resetting efficiency similar to miR-98-5p. Similarly, resetting efficiency was confirmed when the transfection period was on Day 2, Day 3 or Day 4 of Fig. 2 (not shown). The resetting efficiency with transfection of the let-7 miRNA family during these periods was lower than the resetting efficiency when transfection was on Day 1, but still higher than the resetting efficiency with "chemical" when culturing was in VPA-containing cRM-1 without transfection with miRNA mimic or ctrl_mimic.

Fig. 5 shows resetting efficiency for expression of all 22 genes in H9EOS, resetting efficiency for transfection with tagBFP, and resetting efficiency without transfection. Culturing was with addition of VPA to cRM-1 in all cases. Even with introduction of tagBFP expression plasmid to confirm the effect of transfection, the resetting efficiency tended to be higher than without transfection, although the increase was not a significant difference. However, the resetting efficiency was increased with introduction of the 22 genes. The reason for the increased percentage with introduction of tagBFP alone is conjectured to be that the total cell count was decreased due to the toxicity of transfection, whereas proliferation of the naive cells resulted in preferential decrease in the denominator of the ratio [naive cells/total cells].

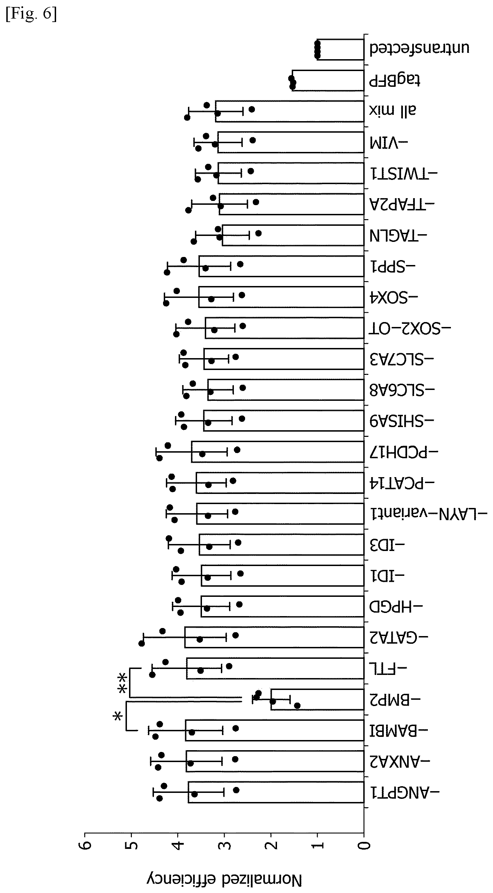

Fig. 6 shows values for resetting efficiency by transfection of H9EOS on Day 1 of Fig. 2 using the 22 different vectors used in Fig. 5 but with the exception of one each, in cRM-1 containing VPA, the values being standardized to resetting efficiency without transfection. From Fig. 6 it was confirmed that the resetting efficiency (standardized value) significantly decreased when hBMP2 was omitted.

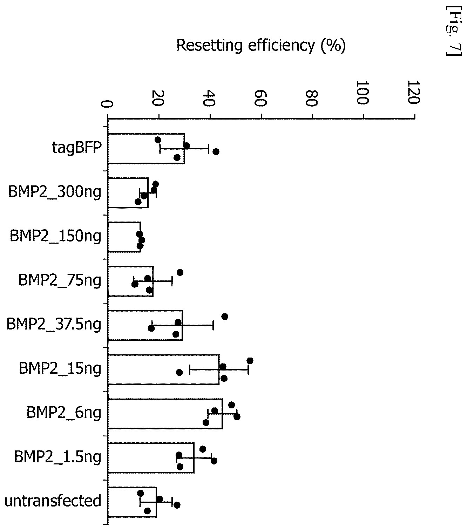

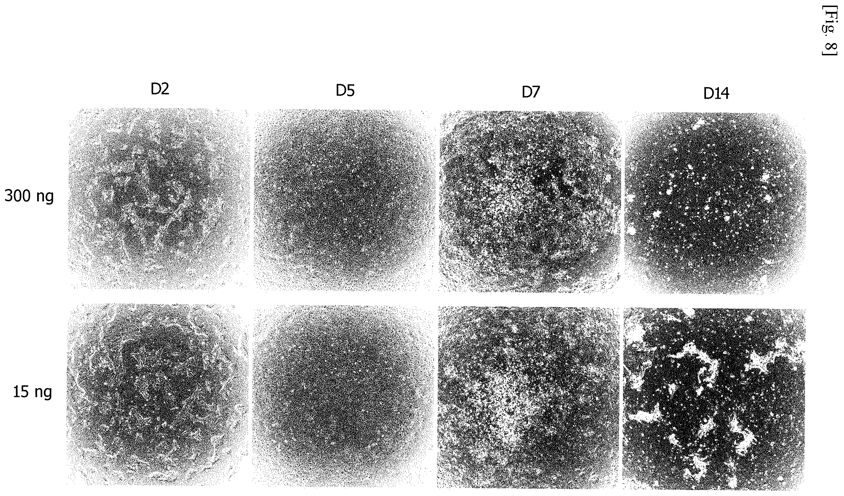

Fig. 7 shows resetting efficiency with different amounts of introduction of a plasmid vector encoding hBMP2 into H9EOS, on Day 1 of Fig. 2, with addition of VPA to cRM-1. Fig. 8 is a set of phase contrast images for introduction of an amount of 300 ng (top row) and 15 ng (bottom row) of plasmid vector encoding hBMP2 into H9EOS, on Day 2, Day 5, Day 7 and Day 14. The resetting efficiency peaked at an hBMP2 expression plasmid introduction amount of about 15 ng. With excess hBMP2, virtually all of the cells died by Day 14.

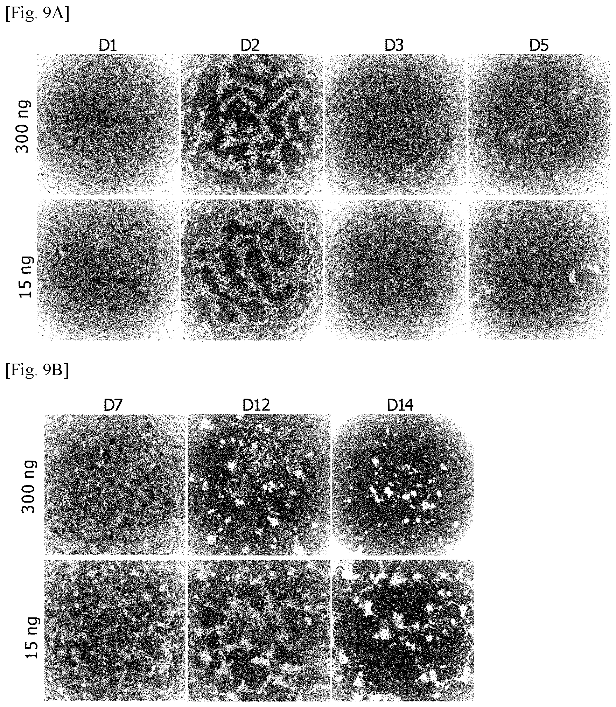

Fig. 9A and Fig. 9B are sets of phase contrast images for introduction of an amount of 300 ng (top row) and 15 ng (bottom row) of plasmid vector encoding hBMP2 into H9EOS, on Day 1, Day 2, Day 3, Day 5, Day 7, Day 12 and Day 14. With introduction of hBMP2 at 300 ng, virtually all of the cells died by Day 14, with essentially no EGFP-positive cells being observed.

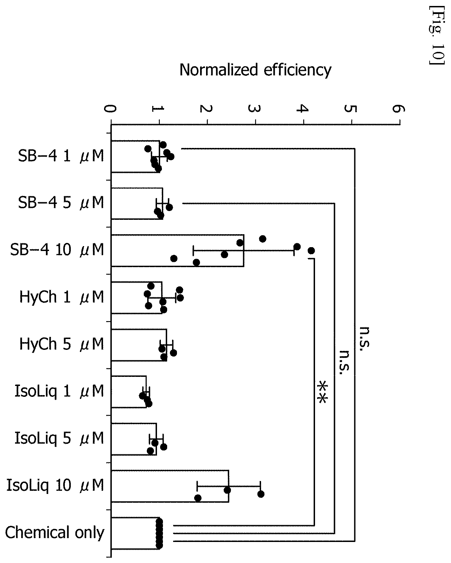

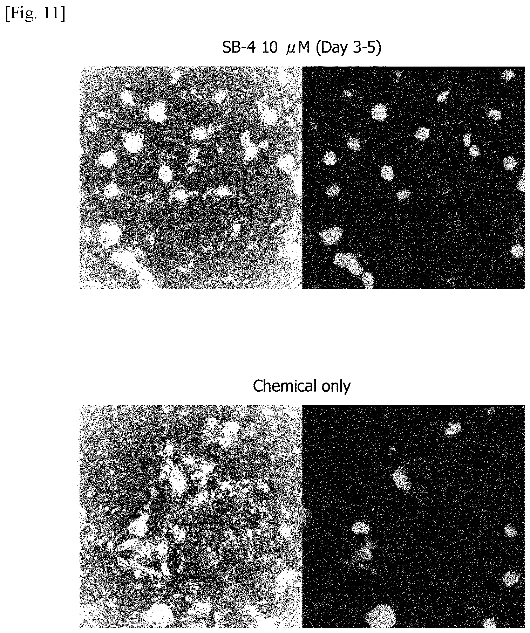

Fig. 10 shows values for resetting efficiency with addition of SB-4, 4-hydroxychalcone (HyCh) and isoliquiritigenin (IsoLiq) at 1, 5 or 10 μM each to the medium, and with addition of VPA to cRM-1, standardized to resetting efficiency of H9EOS without small molecule addition and with culturing in VPA-containing cRM-1 (Chemical only), on Days 3-5 of Fig. 2. Fig. 11 shows a phase contrast image (top left) and a fluorescent image (top right) for Day 14, when SB-4 was added at 10 μM to the medium on Days 3 to 5 of Fig. 2, and a phase contrast image (bottom left) and fluorescent image (bottom right) of H9EOS on Day 14 without addition of a small molecule such as SB-4 (Chemical only). Based on Figs. 10 and 11, it was confirmed that SB-4 and isoliquiritigenin are small molecular compounds capable of activating BMP.

Fig. 12 shows the difference in resetting efficiency with culturing in cRM-1(VPA+) containing VPA or cRM-1(VPA-) containing no VPA according to the protocol shown in Fig. 2, depending on substance transfected into H9EOS, or on substance added to the medium or timing of the addition. Here "chemical" indicates that resetting was carried out without addition of miR-98-5p, ctrl_mimic or BMP. Based on Fig. 12 addition of BMP2 or BMP4 on Day 2 during culturing using VPA-containing cRM-1 showed a highly significant increase in resetting efficiency compared to "chemical". Transfection of miR-98-5p on Day 1 under these conditions tended to further increase the resetting efficiency. Moreover, an increase in resetting efficiency with respect to "chemical" was observed even with culturing using VPA-free cRM-1, when miR-98-5p was transfected on Day 1 and BMP2 or BMP4 was added on Day 2, confirming that naive cells were induced by chemical resetting without using an HDAC inhibitor.

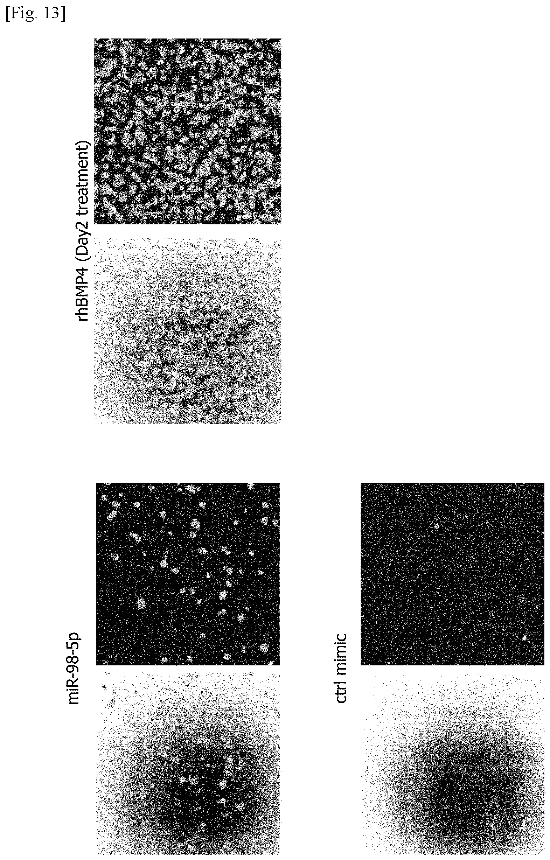

Fig. 13 shows phase contrast images (left panels) and fluorescent images (right panels) of H9EOS resetting efficiencies on Day 14, for transfection of miR-98-5p or ctrl_mimic into H9EOS on Day 1 of Fig. 2, and for addition of rhBMP4 or rhBMP2 to the medium on Day 2. Figs. 12 and 13 confirmed that the highest resetting efficiency is exhibited when the timing of addition of recombinant bone morphogenetic protein to the medium is at the start of culturing in culture medium for naive pluripotent stem cells. On the other hand, the resetting efficiency was lower with addition of rhBMP4 on Day 0. As the reason for this it is conjectured that since allowing rhBMP4 to act on ordinary iPS cells begins to induce mesoderm formation, differentiation to the mesoderm begins before resetting by addition of VPA-containing cRM-1, resulting in loss of pluripotency. Since pluripotency is lost after being coaxed toward differentiation, it is thought that resetting does not take place even with addition of VPA-containing cRM-1. Mesoderm differentiation and resetting are both actions exhibited by rhBMP4, and it is believed that the presence or absence of VPA determines progression toward either differentiation or dedifferentiation (resetting).

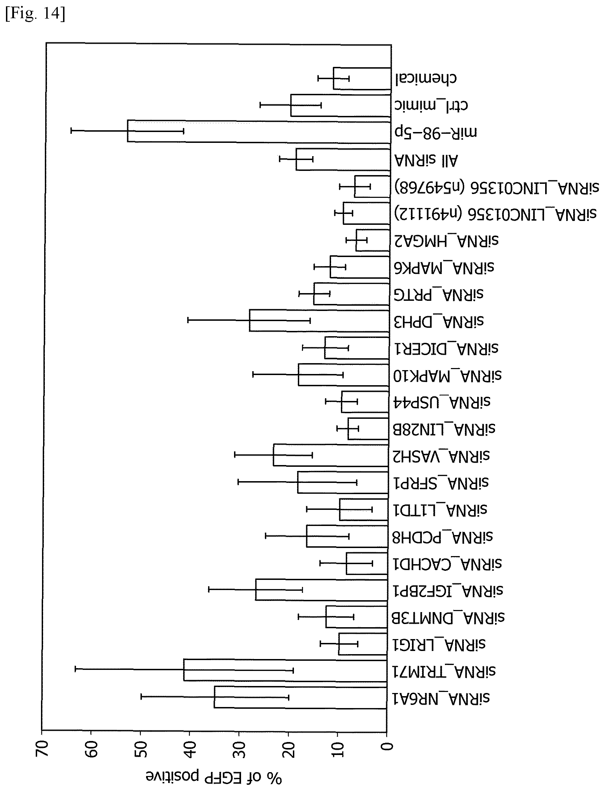

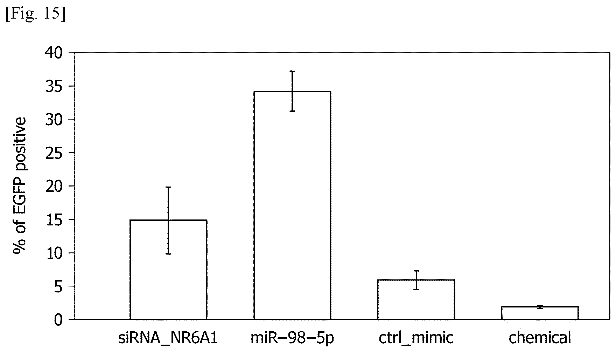

Fig. 14 shows the percentage of EGFP-positive cells resulting by knockdown of specific proteins by siRNA to suppress protein activity (expression). Mimic N.C. represents "negative control mimic", unrelated to any of the miRNAs. When activity was suppressed by knockdown of NR6A1 and TRIM71, the resetting efficiency was significantly increased compared to culturing in VPA-containing cRM-1 without knockdown (chemical). The IGF2BP1, VASH2 and DPH3 knockdowns also showed a tendency toward increased resetting efficiency compared to the control (ctrl_mimic). Fig. 15 shows the results of detailed examination of NR6A1 using siRNA. Knockdown of NR6A1 by siRNA increased resetting efficiency compared to "chemical".

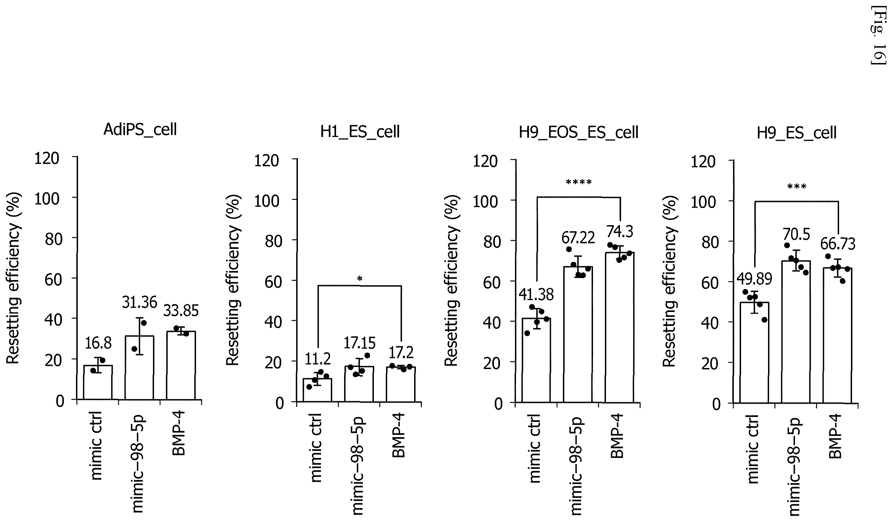

Fig. 16 is a graph showing resetting efficiency in culturing with VPA-containing cRM-1, using AdiPS iPS cells and H1, H9EOS and H9 ES cells. With mimic_ctrl and mimic-98-5p, cells were transfected with control mimic or miR-98-5p mimic on Day 1, culturing was carried out in VPA-containing cRM-1 from Day 2 to Day 4, and culturing was in cRM-2 from Day 5 to Day 14. For BMP-4, the BMP4 was added to VPA-containing cRM-1 on Day 2 without transfection of cells with control mimic or miR-98-5p mimic, for culturing up to Day 4, and then culturing was in cRM-2 from Day 5 to Day 14. The cells were detached with Accumax, suspended in 1% BSA-PBS and blocked for 30 minutes on ice, and then SUSD2 antibody solution was mixed with the cell suspension to a final concentration of 1:500, reaction was conducted for 30 minutes on ice, the mixture was rinsed with PBS and measurement was performed by flow cytometry. For all of the cell lines, treatment with miR-98-5p or BMP4 tended to increase the percentage of SUSD2-positive cells.

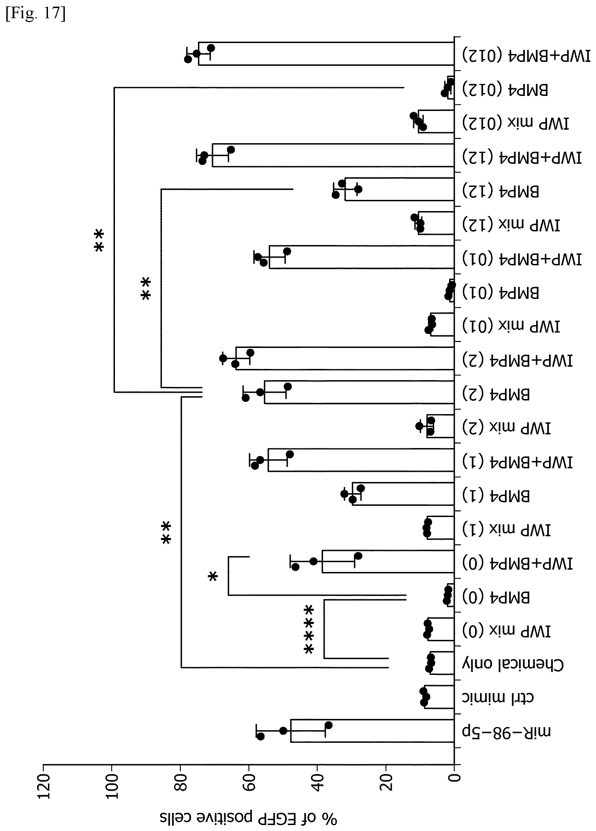

Fig. 17 is a graph showing the effects of BMP and Wnt inhibitor on resetting efficiency when culturing with VPA-containing cRM-1, using H9EOS cells. After seeding H9EOS cells in AK02N (lock inhibitor, containing iMatrix-511) on Day 0 and exchanging the medium with AK02N on Day 1, they were transfected with miR-98-5p using RNAiMax. The medium was exchanged with VPA-containing cRM-1on Day 2 and with cRM-2 on Day 5, continuing culturing in cRM-2 until Day 14, after which the GFP-positive cells were counted. BMP4 and/or IWP mix was added to the medium at one or more time points of Day 0, Day 1 and Day 2. The IWP mix was a cocktail of IWP-2, IWP-3 and IWP-4, added in amounts of 1 μM. In the graph, IWP+BMP4 represents addition of IWP mix and BMP4 to the medium simultaneously. When Wnt inhibitor was added to the medium either simultaneously with or before BMP4, it was possible to add BMP4 at an earlier time point while maintaining the resetting efficiency, and high resetting efficiency was even obtained with addition of BMP during the culturing step in culture medium for primed pluripotent stem cells.

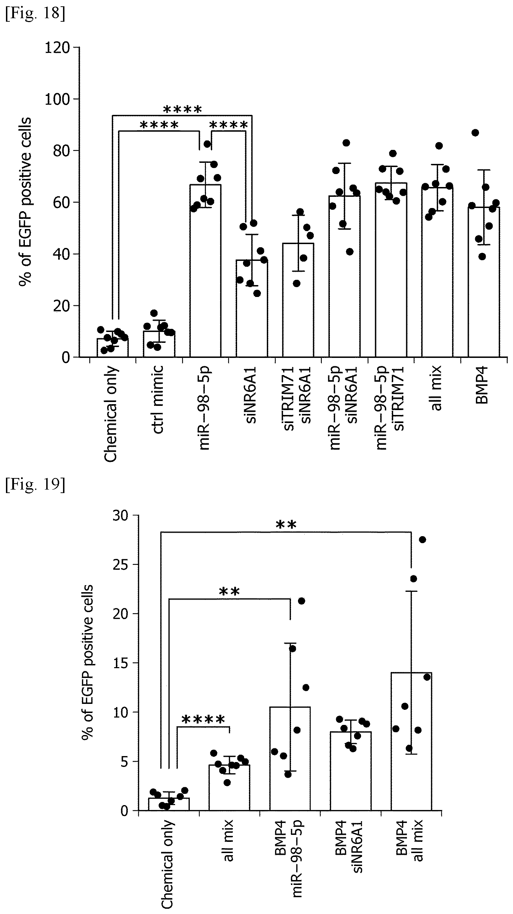

Fig. 18 is a graph showing resetting efficiency when culturing with VPA-containing cRM-1, using H9EOS cells. After seeding H9EOS cells in AK02N (lock inhibitor, containing iMatrix-511) on Day 0 and exchanging the medium with AK02N on Day 1, they were transfected with different siRNAs or miR-98-5p using RNAiMax. The medium was exchanged with VPA-containing cRM-1 on Day 2. The BMP4-treated samples had BMP4 already added to the cRM-1 medium of Day 2. The medium was exchanged with cRM-2 on Day 5, continuing culturing in cRM-2 until Day 14, after which the GFP-positive cells were counted. In Fig. 18, "all mix" represents a mixture of miR-98-5p, siNR6A1 and siTRIM71. The reset miRNA, reset siRNA and BMP were all able to drastically increase resetting efficiency even with each alone.

Fig. 19 is a graph showing resetting efficiency when culturing with VPA-free cRM-1, using H9EOS cells. The protocol was the same as in Fig. 18, except that VPA-free cRM-1 was used instead of VPA-containing cRM-1. In Fig. 19, "Chemical only" represents the results of culturing in medium without VPA, and without reset miRNA, reset siRNA or BMP, while "all mix" represents a mixture of miR-98-5p, siNR6A1 and siTRIM71. Resetting efficiency could be increased with reset miRNA, reset siRNA and/or BMP, even with culturing in VPA-free medium.

The results of a knockdown experiment for the miR-98-5p targets NR6A1 and TRIM71 demonstrated that in production of naive pluripotent stem cells, NR6A1 knockdown promotes induction from primed pluripotent stem cells to naive pluripotent stem cells, while TRIM71 knockdown removes cells differentiated to other than naive pluripotent stem cells (data not shown). Moreover, the results of an experiment with overexpression of NR6A1 confirmed that NR6A1 inhibits induction of primed pluripotent stem cells to naive pluripotent stem cells (data not shown). In addition, results of an experiment with addition of BMP inhibitor showed reduced induction efficiency of naive pluripotent stem cells, indicating that the BMP signal promotes induction of naive pluripotent stem cells (data not shown).

[Sequence Listing]

**Claims**

1. A method for producing a naive pluripotent stem cell, comprising the following steps:

   (1) a step of culturing a primed pluripotent stem cell in culture medium for primed pluripotent stem cells; and
   (2) a step of culturing the cell obtained in step (1) in culture medium for naive pluripotent stem cells,

   and comprising either or both of the following steps:

   (i) a step of introducing miRNA selected from among miRNA belonging to the let-7 miRNA family and their similar sequences into the cell to be cultured in step (1) or (2), or expressing the miRNA in the cell; or
   a step of introducing an activity inhibitor for a target protein of the let-7 miRNA family into the cell to be cultured in step (1) or (2), or expressing the activity inhibitor in the cell; and
   (ii) a step of introducing bone morphogenetic protein into the cell to be cultured in step (1) or (2) and expressing the bone morphogenetic protein in the cell, or adding the bone morphogenetic protein to the medium of step (1) or (2); or

   a step of adding a bone morphogenetic protein activator to the medium of step (1) or (2).

2. The method according to claim 1, further comprising a step of adding Wnt inhibitor to the medium of step (1) or (2) either before or simultaneously with step (ii).

3. The method according to claim 1, comprising (A) step (i), in which the culture medium for naive pluripotent stem cells of step (2) does not comprise a histone deacetylase inhibitor.

4. The method according to claim 3, further comprising step (ii).

5. The method according to claim 1, comprising (B) step (i) or step (ii), in which the culture medium for naive pluripotent stem cells of step (2) comprises a histone deacetylase inhibitor.

6. The method according to claim 1, wherein the histone deacetylase inhibitor comprises valproic acid.

7. The method according to claim 1, wherein in step (i), the miRNA belonging to the let-7 miRNA family or a similar sequence thereof is selected from among miR-98-5p, let-7a-5p, let-7b-5p, let-7c-5p, let-7e-5p, let-7f-5p, let-7g-5p, let-7i-5p, and miRNA having at least 80% sequence identity to the same.

8. The method according to claim 1, wherein in step (ii), the bone morphogenetic protein is selected from among BMP2 and BMP4.

9. The method according to claim 5, wherein in step (ii), the bone morphogenetic protein activator is selected from among SB-4 and isoliquiritigenin.

10. The method according to claim 1, wherein in step (i), the target protein of miRNA belonging to the let-7 miRNA family is NR6A1 and/or TRIM71.

11. The method according to claim 1, further comprising step (3) in which the cell obtained in step (2) is additionally cultured in culture medium for naive pluripotent stem cells containing Tankyrase/Wnt/β-catenin inhibitor and PKC inhibitor.

12. The method according to claim 11, further comprising a step in which the cell obtained in step (3) is subcultured in culture medium for naive pluripotent stem cells according to claim 11.

[Fig. 1]

[Fig. 2]

[Fig. 3]

[Fig. 4]

[Fig. 5]

[Fig. 6]

[Fig. 7]

[Fig. 8]

[Fig. 9A]

D1　　　D2　　　D3　　　D5

300 ng

15 ng

[Fig. 9B]

D7　　　D12　　　D14

300 ng

15 ng

[Fig. 10]

[Fig. 11]

SB-4 10 $\mu$M (Day 3-5)

Chemical only

[Fig. 12]

[Fig. 13]

[Fig. 14]

[Fig. 15]

[Fig. 16]

[Fig. 17]

[Fig. 18]

[Fig. 19]

## INTERNATIONAL SEARCH REPORT

| International application No. |
| --- |
| **PCT/JP2023/031853** |

**A.    CLASSIFICATION OF SUBJECT MATTER**

*C12N 5/0735*(2010.01)i; *C12N 5/10*(2006.01)i
FI:    C12N5/0735; C12N5/10 ZNA

According to International Patent Classification (IPC) or to both national classification and IPC

**B.    FIELDS SEARCHED**

Minimum documentation searched (classification system followed by classification symbols)

C12N5/0735; C12N5/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2022-534395 A (CHILDREN'S HOSPITAL MEDICAL CTR.) 29 July 2022 (2022-07-29) <br> particularly, claims 1-66, paragraph [0074], examples | 1-12 |
| Y | SANG, H. et al. Dppa3 is critical for Lin28a-regulated ES cells naive-primed state conversion. Journal of Molecular Cell Biology. 2019, vol. 11, pp. 474-488 <br> particularly, abstract, p. 483, right column, second paragraph to p. 484, left column, first paragraph, fig. 6, 7 | 1-12 |
| Y | YU, S. et al. BMP4 drives primed to naive transition through PGC-like state. Nature Communications. 19 May 2022, vol. 13, no. 2756, pp. 1-15 <br> particularly, abstract, p. 2, left column, second paragraph to fifth paragraph, fig. 1 | 1-12 |
| A | LI, M. A. et al. A lncRNA fine tunes the dynamics of a cell state transition involving Lin28, let-7 and de novo DNA methylation. Elife. 2017, vol. 6, no. e23468, pp. 1-24 <br> entire text | 1-12 |
| A | WO 2019/093340 A1 (KYOTO UNIV.) 16 May 2019 (2019-05-16) <br> entire text | 1-12 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **02 November 2023** | **14 November 2023** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
| --- |
| **PCT/JP2023/031853** |

**Box No. I**     **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed:

   ☑ in the form of an Annex C/ST.25 text file.

   ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

   ☐ in the form of an Annex C/ST.25 text file (Rule 13*ter*.1(a)).

   ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "In the form of an Annex C/ST.25 text file" above should be understood as "in ST.26 format".

Form PCT/ISA/210 (continuation of first sheet) (January 2015)

EP 4 582 534 A1

**INTERNATIONAL SEARCH REPORT**
Information on patent family members

International application No.

**PCT/JP2023/031853**

| Patent document cited in search report | | | Publication date (day/month/year) | Patent family member(s) | Publication date (day/month/year) |
|---|---|---|---|---|---|
| JP | 2022-534395 | A | 29 July 2022 | WO 2020/243615 A1 particularly, claims 1-66, paragraph [0115], examples | |
| WO | 2019/093340 | A1 | 16 May 2019 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050209261
- US 20050192304 A
- US 20040014755 A
- US 20040002508 A
- US 20040002507 A
- US 20030125344 A
- US 20030087919 A
- WO 2003062227 A
- WO 2003059913 A
- WO 2003062225 A
- WO 2002076976 A
- WO 2004039796 A

**Non-patent literature cited in the description**

- **TAKASHIMA et al.** *Cell*, 2014, vol. 158, 1254-1269
- **GUO et al.** *Development.*, 2017, vol. 144, 2748-2763
- **SZCZERBINSKA et al.** *Stem Cell Rep.*, 2019, vol. 13, 612-626
- *Scientific Report*, 2014, vol. 4
- **ISHIZAKI et al.** *Mol. Pharmacol.*, 2000, vol. 57, 976-983
- **NARUMIYA et al.** *Methods Enzymol.*, 2000, vol. 325, 273-284
- **UENATA et al.** *Nature*, 1997, vol. 389, 990-994
- **SASAKI et al.** *Pharmacol. Ther.*, 2002, vol. 93, 225-232
- **NAKAJIMA et al.** *Cancer Chemother. Pharmacol.*, 2003, vol. 52 (4), 319-324
- *CHEMICAL ABSTRACTS*, 391210-10-9
- *CHEMICAL ABSTRACTS*, 109511-58-2
- *CHEMICAL ABSTRACTS*, 167869-21-8
- *CHEMICAL ABSTRACTS*, 212631-79-3
- *CHEMICAL ABSTRACTS*, 252917-06-9
- *CHEMICAL ABSTRACTS*, 667463-62-9
- *CHEMICAL ABSTRACTS*, 142273-20-9
- *CHEMICAL ABSTRACTS*, 1129669-05-1
- *CHEMICAL ABSTRACTS*, 133053-19-7
- *CHEMICAL ABSTRACTS*, 133052-90-1
- *CHEMICAL ABSTRACTS*, 284028-89-3
- *CHEMICAL ABSTRACTS*, 1404559-91-6
- *CHEMICAL ABSTRACTS*, 1404562-17-9
- *CHEMICAL ABSTRACTS*, 100874-08-6